# EUROPEAN PATENT APPLICATION

(11) **EP 2 324 815 A1**
(43) Date of publication of application: **25.05.2011**
(21) Application number: 10197470.7
(22) Date of filing: 16.11.2006
(51) Int. Cl.: A61K 8/26, A61K 8/28, A61K 8/44, A61K 8/368, A61Q 15/00

(54) **Antiperspirant compositions**

(30) Priority: 16.11.2005 US 737207 P
(62) Divisional of application: 06846328.0
(71) Applicant: Colgate-Palmolive Company, New York, NY 10022 (US)
(72) Inventor: Mattai, Jairajh, Piscataway, NJ 08854 (US); Kilpatrick-Liverman, Latonya, Princeton, NJ 08542 (US); Holerca, Marian, Somerset, NJ 08873 (US); Tang, Xiozhong, Cherry Hill, NJ 08003 (US)
(74) Representative: Jenkins, Peter David

(57) **Abstract**

An antiperspirant composition comprising a mixing product of at least one salt chosen from at least one aluminum salt, at least one aluminum-zirconium salt, at least one aluminum salt complex, and at least one aluminum-zirconium salt complex; at least one cosmetically acceptable hydroxy acid; and at least one cosmetically acceptable quaternary ammonium acid compound.

## Description

### CROSS REFERENCE TO RELATED APPLICATION

This application claims priority to U.S. Provisional Patent Application No. 60/737,207, filed on 16 November 2005, which is incorporated herein by reference.

### BACKGROUND OF THE INVENTION

Antiperspirant compositions containing aluminum or aluminum-zirconium salts tend to exhibit polymerization of these salts over time, forming species with molecular weights ranging from about 500 to about 500,000 g/mol. In general, lower molecular weight species have greater antiperspirant effect than higher molecular weight species. Without being bound by theory, it is believed that the smaller molecules more readily and more effectively occlude sweat pores, thereby producing the desired antiperspirant effect. Thus, reducing the size of the polymers enhances the antiperspirant effect and moreover lowers the amount of antiperspirant salt that is necessary to control perspiration. The ability to reduce the amount of antiperspirant salt in a formulation without compromising efficacy would bring several advantages. Antiperspirant salts are a relatively expensive component of a typical antiperspirant formulation. It is thus very desirable to have compositions wherein antiperspirant salts are stabilized to reduce polymerization.

The extent of antiperspirant polymerization can be measured by size exclusion chromatography (SEC), also known as gel filtration chromatography (GFC). Passage of small molecules through an SEC column is retarded while large molecules pass through more rapidly. Elution time of a polymeric substance migrating through the SEC column is correlated with the size of the polymer molecules, and this relationship allows the apparent molecular weight of a polymer to be determined. In most cases, 4 to 6 well defined groups of polymerized species in aluminum or aluminum-zirconium salt compositions can be identified by SEC and are commonly known as peaks 1, 2, 3, 4, 5, and 6 (6 is not always present). Peak 1 is associated with zirconium species and so is present only for aluminum-zirconium salts. Peak 2 is not always present in the case of aluminum-zirconium salts. Peak 6 is not always present. The earlier peaks (1, 2, and 3) correspond to the larger species and the later peaks (4, 5 and 6) correspond to the smaller and more desirable species. Peaks correlate with weight average values of molecular weight for polymers, not as discrete values.

There remains a need in the art for improved antiperspirant and/or deodorant compositions. In particular, it would be desirable to provide such compositions having improved stability. It would further be desirable to provide such compositions having enhanced antiperspirant efficacy. It would still further be desirable to provide such compositions wherein formation of higher molecular weight zirconium and/or aluminum polymeric species is reduced or suppressed, particularly where such reduction or suppression leads to enhanced efficacy, for example related to more effective occlusion of sweat pores.

### SUMMARY OF THE INVENTION

An antiperspirant composition comprising a mixing product of:
(a) at least one salt chosen from at least one aluminum salt, at least one aluminum-zirconium salt, at least one aluminum salt complex, and at least one aluminum-zirconium salt complex;
(b) at least one cosmetically acceptable hydroxy acid; and
(c) at least one cosmetically acceptable quaternary ammonium acid compound.

### DETAILED DESCRIPTION OF THE INVENTION

As used throughout, ranges are used as a shorthand for describing each and every value that is within the range. Any value within the range can be selected as the terminus of the range.

In one embodiment, the antiperspirant composition comprises a mixing product of:
(a) an aluminum and/or aluminum-zirconium salt or a complex thereof;
(b) a cosmetically acceptable hydroxy acid, and
(c) a cosmetically acceptable quaternary ammonium acid compound.

The components of antiperspirant composition may be present in free or salt form, and depending on the formulation, may be precipitated or dissolved or in the form of solvates.

The aluminum or aluminum-zirconium salts may be present for example in chloride or chlorohydrate form, *e.g*., aluminum chloride, aluminum chlorohydrate, aluminum sequichlorohydrate, aluminum dichlorohydrate, aluminum zirconium tricchlorohydrate, aluminum zirconium tetrachlorohydrate, aluminum zirconium pentachlorohydrate, aluminum zirconium octachlorohydrate, or mixtures thereof. Optionally, these salts may form or be provided as complexes with other compounds, for example complexes with the hydroxy acids and/or quaternary ammonium acids as described, and/or amino acids, such as glycine, and/or polyhydric alcohols, *e.g*., ethylene glycol, polyethylene glycol, propylene glycol, or polypropylene glycol. When the antiperspirant comprises an aluminum-zirconium salt, the salt may, for example, have a molar ratio of aluminum to zirconium of about 2 to about 10, for example, about 2 to about 5, or about 5 to about 10. In some embodiments comprising an aluminum-zirconium salt, the aluminum-zirconium salt may for example be an aluminum-zirconium chlorohydrate salt or complex thereof, wherein the molar ratio of (Aluminum and Zirconium) to chloro atoms is about 0.9 to about 2.1, for example, about 1.2 to about 1.8.

The quaternary ammonium acid compound typically exists in zwitterionic form, forming an inner salt between cationic quaternary ammonium portion and the anionic carboxylic acid portion. It may however be present in salt form, *e.g*., acid addition salt form. For example betaine may be provided in the form of betaine hydrochloride.

The divalent metal cation may be provided in salt or oxide form, for example calcium may be provided in the form of, *e.g*., calcium chloride or calcium oxide.

In one embodiment of the antiperspirant composition, the antiperspirant salt is an aluminum-zirconium chlorohydrate salt and the quaternary ammonium acid is betaine, wherein the metal (Aluminum and Zirconium) to chloride ratio is about 0.9 to about 2.1, the betaine to zirconium ratio is about 0.1 to about 2, and the betaine to hydroxy acid ratio is about 0.2 to about 30.

In one embodiment, the composition exhibits improved stability compared to analogous formulations lacking a hydroxy acid or lacking a hydroxy acid and a divalent cation, *e.g*., as measured by at least one of (a) a reduction in formation of higher molecular weight polymerized aluminum and/or zirconium species as indicated at least by reduction in area of SEC peaks 2 and/or 3 and/or increase in area of SEC peaks 4 and/or 5; (b) a reduction in formation of higher molecular weight polymerized zirconium species as indicated at least by reduction in area of SEC peak 1; or (c) an increase in peak 5 as compared to peak 4. For example, compositions when analyzed by SEC as a 5% aqueous solution using conditions capable of resolving the Aluminum and/or Zirconium species into at least 4 successive peaks, in one embodiment, exhibit a ratio of the SEC area of the first major peak (peak 1, largest polymer) to the SEC area of the last major peak (usually peak 5) of less than about 1, and in another embodiment of less than about 0.25.

There is further provided a method for controlling perspiration, the method comprising applying to skin an antiperspirant effective amount of the antiperspirant compositions as described above.

There is still further provided a process for preparing a stabilized antiperspirant composition, or for stabilizing an aluminum or aluminum zirconium antiperspirant salt or complex thereof, the process comprising:
(i) mixing at least one aluminum salt, at least one aluminum-zirconium salt, at least one aluminum salt complex, and/or at least one aluminum-zirconium salt complex with at least one quaternary ammonium acid component in an aqueous medium; and
(ii) adding to the resulting mixture at least one hydroxy acid (and optionally a divalent cation) to form the composition.

In other embodiments, there is provided antiperspirant compositions obtained or obtainable by the foregoing process.

In another embodiment, there is provided an antiperspirant and/or deodorant formulation for topical use, comprising at least one stabilized antiperspirant compositions in combination with a cosmetically acceptable carrier. Such formulations may be as known in the art, *e.g*., in the form of a stick, gel, cream, liquid roll-on, or aerosol spray. Examples of formulations comprising the stabilized antiperspirant compositions of the invention are provided below.

SEC peak areas are used as indicators of aluminum and zirconium polymer size in antiperspirant salt solutions. It is believed, without being bound by theory, that enhanced antiperspirant and/or deodorant efficacy is associated with increase in peak 4 and/or peak 5 area relative to peak 2 and/or peak 3 area, and with decrease in peak 1 area, indicating a lower degree of polymerization of the aluminum and zirconium species respectively. Relative areas of peaks 1 through 5 constitute the "SEC profile", as that term is used herein, of an antiperspirant and/or deodorant composition. Stabilized compositions of the invention maintain, over a period of time, improved SEC profiles, indicating a lower degree of polymerization of the aluminum and/or zirconium species after aging, by comparison with non-stabilized compositions, for example compositions lacking a calcium salt and/or a hydroxy acid.

Peak areas can be adjusted based on an internal standard, with total aluminum peak area (sum of peaks 2 through 5) as the common denominator. This is possible as total aluminum peak area remains constant. Adjusting peak areas to an internal standard in this fashion better reflects the polymer distribution in the sample.

In certain embodiments, the relative amounts of components (a), (b), and (c) of the antiperspirant composition as described above can be such that an aqueous solution of these components in the same relative amounts, at a 25% concentration of (a) + (b), following aging of the solution at about 45°C for about 60 days, has an adjusted SEC peak 1 area of no more than about 40%, for example no more than about 20%, or no more than about 10%.

Illustratively, the relative amounts of components (a), (b), and (c) as described above are such that an aqueous solution of these components in the same relative amounts, at a 25% concentration of (a) + (b), following aging of the solution at about 45°C for about 60 days, has an adjusted SEC peak 5 area of at least about 30%, for example at least about 40%.

In certain embodiments, the relative amounts of components (a), (b), and (c) as described above are such that an aqueous solution of these components in the same relative amounts, at a 25% concentration of (a) + (b), following aging of the solution at about 45°C for about 60 days, has an adjusted SEC peak 4 area of at least about 15%. The adjusted SEC peak 4 area is optionally not more than about 25%.

In the composition according to one embodiment of the invention, the relative amounts of components (a), (b), and (c) as described above are such that an aqueous solution of these components in the same relative amounts, at a 25% concentration of (a) + (b), following aging of the solution at about 45°C for about 60 days, has:
(i) an adjusted SEC peak 1 area of no more than about 40%, for example no more than about 20%, or no more than about 10%;
(ii) an adjusted SEC peak 5 area of at least about 30%, for example at least about 40%; and
(iii) an adjusted SEC peak 4 area of at least about 15%.

In certain embodiments, the aluminum or aluminum-zirconium salt of the antiperspirant compositions of the invention can be present in the final topical formulation in any suitable total amount, typically about 4% to about 35%, for example about 10% to about 25%, or about 15% to about 20%, by weight of the formulation. It will be understood that while amounts in various ranges of aluminum salts, aluminum-zirconium salts or complexes thereof are indicated herein, lesser amounts can be used to contribute to deodorant activity of deodorant products which are not classified as antiperspirants.

The antiperspirant compositions of the invention also comprise a quaternary ammonium acid compound, *e.g*., an alpha-quaternary ammonium-carboxylic acid, for example, betaine. Betaine is 1-carboxy-N,N,N-trimethylmethanaminium hydroxide inner salt (IUPAC nomenclature), and is zwitterionic. It occurs naturally in many foodstuffs and can also be synthesized. Though sometimes incorrectly referred to as an amino acid, it will be understood that betaine is not truly an amino acid. A "betaine component" herein can be betaine or a salt thereof. In certain embodiments, the betaine component comprises betaine, betaine hydrochloride or a mixture thereof. The betaine component may, for example, be present in the final formulation for topical use in a total amount of about 0.5 to about 20% for example about 5% to about 15%, by weight. In one embodiment, when the composition comprises an aluminum-zirconium salt, the molar ratio of betaine to zirconium may be about 0.1 to about 2, for example, about 0.1 to about 1.

The composition of the invention may also optionally comprise at least one divalent cation, *e.g.* in the form of a salt. In one embodiment, this cation is a calcium salt or oxide, *e.g.,* calcium chloride, calcium bromide, calcium nitrate, calcium acetate, calcium formate, calcium gluconate, calcium ascorbate, calcium lactate, calcium glycinate, calcium citrate, calcium carbonate, calcium oxide, calcium hydroxide, calcium phosphate, calcium phosphonate, calcium picrolonate, calcium sulfate, calcium sulfonate, calcium sulfocyanate, calcium perrhenate or mixtures thereof. In certain embodiments, the calcium salt comprises an anion having a linear or branched C₁₋₆₀ alkyl or alkenyl chain and at least one functional moiety capable of binding a calcium ion. The at least one functional moiety can be a nitrate, sulfate, sulfonate, carbonate, carbonyl, phosphate, phosphonate or hydroxyl moiety. The anion can comprise one or more heteroatoms independently selected from nitrogen, oxygen and sulfur, such heteroatoms being part of the chain and/or part of the functional moiety. In one embodiment, when the divalent cation is provided in the form of a salt, it may be present in the final formulation in a total amount of about 0.2% to about 10%, for example about 2% to about 8%, by weight. In one embodiment, the molar ratio of the divalent metal ion to the aluminum or (Aluminum and Zirconium) may be about 0.02 to about 1.2, for example about 0.1 to about 0.8.

The composition also comprises at least one hydroxy acid. Illustrative hydroxy acids include α-hydroxy acids, β-hydroxy acids and mixtures thereof. In certain embodiments, the hydroxy acid is chosen from lactic acid, glycolic acid, lactobionic acid, carnitine, salicylic acid and mixtures thereof. In certain embodiments, the alpha-hydroxy acid has a pKₐ of about 4.5 or less, about 2.5 to about 4.5, or about 3.5 to about 4.0.

In certain embodiments, the at least one hydroxy acid is present in a total amount of about 0.2% to about 10%, for example about 0.5% to about 9%, or about 2% to about 8%, by weight. In certain embodiments, the molar ratio of quaternary ammonium acid compound, to hydroxy acid is typically about 0.2 to about 30, for example, about 5 to about 25, or about 10 to about 20.

In certain embodiment, the composition has:
(i) a molar ratio of (Aluminum and Zirconium) to halo atoms in the at least one aluminum-zirconium salt or complex thereof of about 0.9 to about 2.1;
(ii) a molar ratio of aluminum to zirconium of about 2 to about 10;
(iii) a molar ratio of calcium to (Aluminum and Zirconium) of about 0.02 to about 1.2;
(iv) a molar ratio of betaine to zirconium of about 0.1 to about 2; and
(v) a molar ratio of betaine to hydroxy acid of about 0.2 to about 30.

Optional ingredients that can be included in an antiperspirant and/or deodorant formulation of the antiperspirant compositions of the invention include solvents; water-soluble alcohols such as C₂₋₈ alcohols including ethanol; glycols including propylene glycol, dipropylene glycol, tripropylene glycol and mixtures thereof; glycerides including mono-, di- and triglycerides; medium to long chain organic acids, alcohols and esters; surfactants including emulsifying and dispersing agents; amino acids including glycine; structurants including thickeners and gelling agents, for example polymers, silicates and silicon dioxide; emollients; fragrances; and colorants including dyes and pigments. If desired, an antiperspirant and/or deodorant agent additional to the aluminum-zirconium salt or complex thereof can be included, for example an odor reducing agent such as a sulfur precipitating agent, *e.g*., copper gluconate, zinc gluconate, zinc citrate, etc.

The antiperspirant compositions can be formulated into topical antiperspirant and/or deodorant formulations suitable for application to skin, illustratively a stick, a gel, a cream, a roll-on, a soft solid, a powder, a liquid, an emulsion, a suspension, a dispersion or a spray. The composition can comprise a single phase or can be a multi-phase system, for example a system comprising a polar phase and an oil phase, optionally in the form of a stable emulsion. The composition can be liquid, semi-solid or solid. The following topical formulations are provided for purposes of exemplification:

In one prophetic example, the formulation is in the form of a spray that comprises:
(a) about 4% to about 25% by weight in total of at least one aluminum-zirconium chlorohydrate salt or complex thereof;
(b) about 0.5 to about 20% by weight in total of at least one betaine component;
(c) about 0.2% to about 10% by weight in total of at least one calcium salt;
(d) about 0.2% to about 10% by weight in total of at least one hydroxy acid;
(e) about 35% to about 87% by weight of water;
(f) about 3% to about 7% by weight in total of at least one water soluble emollient; and
(g) about 0.5% to about 3% by weight in total of at least one cosmetically acceptable surfactant selected from cationic surfactants, nonionic surfactants, anionic surfactants, amphoteric surfactants, dimethicone copolyols, polyether ethoxylates, and mixtures thereof.

In another prophetic example, the formulation is in the form of a roll-on that comprises:
(a) about 4% to about 25% by weight in total of at least one aluminum-zirconium chlorohydrate salt or complex thereof;
(b) about 0.5 to about 20% by weight in total of at least one betaine component;
(c) about 0.2% to about 10% by weight in total of at least one calcium salt;
(d) about 0.2% to about 10% by weight in total of at least one hydroxy acid;
(e) about 27% to about 88% by weight of water;
(f) about 0.5% to about 3% by weight in total of at least one magnesium aluminum silicate;
(g) about 0.5% to about 10% by weight in total of at least one cosmetically acceptable surfactant selected from cationic surfactants, nonionic surfactants, anionic surfactants, amphoteric surfactants, dimethicone copolyols, polyether ethoxylates, and mixtures thereof; and
(h) 0% to about 5% by weight in total of at least one water miscible solvent.

In certain embodiments, the antiperspirant composition is present in the formulation in a polar phase, and the formulation further comprises an oil phase.

In another prophetic example, the formulation is in the form of a stick, wherein the polar phase comprises, by weight of the formulation:
(a) about 4% to about 30% in total of at least one aluminum-zirconium chlorohydrate salt or complex thereof;
(b) about 0.5% to about 20% in total of at least one betaine component;
(c) about 0.2% to about 10% in total of at least one calcium salt;
(d) about 0.2% to about 10% in total of at least one hydroxy acid;
(e) about 5% to about 40% in total of water, one or more water miscible solvents, or a mixture thereof; and
(f) 0% to about 5% in total of at least one cosmetically acceptable surfactant selected from cationic surfactants, nonionic surfactants, anionic surfactants, amphoteric surfactants, dimethicone copolyols, polyether ethoxylates, and mixtures thereof;
   and the oil phase comprises, by weight of the formulation:
(g) about 0.5% to about 8.0% of a siloxane polyamide gelling agent;
(h) about 20% to about 60% of a volatile organic or silicone based fluid;
(i) 0% to about 20% in total of at least one cosmetic ingredient selected from C₈₋₂₂ fatty alcohols, C₁₂₋₃₆ fatty esters, C₈₋₁₈ alkyl benzoates, linear polysiloxanes, and mixtures thereof;
(j) 0% to about 10% in total of at least one cosmetically acceptable surfactant selected from cationic surfactants, nonionic surfactants, anionic surfactants, amphoteric surfactants, dimethicone copolyols, polyether ethoxylates, and mixtures thereof; and
(k) 0% to about 3% of a fragrance.

In another prophetic example, the formulation is in the form of a stick, wherein the polar phase comprises, by weight of the formulation:
(a) about 4% to about 30% in total of at least one aluminum-zirconium chlorohydrate salt or complex thereof;
(b) about 0.5 to about 20% in total of at least one betaine component;
(c) about 0.2% to about 10% in total of at least one calcium salt;
(d) about 0.2% to about 10% in total of at least one hydroxy acid;
(e) about 5% to about 40% in total of water, one or more water miscible solvents, or a mixture thereof; and
(f) 0% to about 5% in total of at least one cosmetically acceptable surfactant selected from cationic surfactants, nonionic surfactants, anionic surfactants, amphoteric surfactants, dimethicone copolyols, polyether ethoxylates, and mixtures thereof; and the oil phase comprises, by weight of the formulation:
(g) about 20% to about 60% in total of one or more cosmetically acceptable solvents selected C₂₋₈ polyhydric alcohols, C₈₋₂₂ unsaturated fatty alcohols, branched and straight chain C₈₋₂₂ saturated fatty alcohols, and mixtures thereof;
(h) 0% to about 10% in total of at least one cosmetically acceptable surfactant selected from cationic surfactants, nonionic surfactants, anionic surfactants, amphoteric surfactants, dimethicone copolyols, polyether ethoxylates, and mixtures thereof;
(i) 0% to about 3% of a fragrance; and
(j) about 5% to about 25% by weight of a linoleic acid dimer based polyamide.

In another prophetic example, the formulation is in the form of a gel, wherein the polar phase comprises, by weight of the formulation:
(a) about 4% to about 35% in total of at least one aluminum-zirconium chlorohydrate salt or complex thereof;
(b) about 0.5 to about 20% in total of at least one betaine component;
(c) about 0.2% to about 10% in total of at least one calcium salt;
(d) about 0.2% to about 10% in total of at least one hydroxy acid;
(e) about 25% to about 60% of water;
(f) about 5% to about 40% in total of at least one water miscible solvent; and
(g) 0% to about 5% in total of at least one cosmetically acceptable surfactant selected from cationic surfactants, nonionic surfactants, anionic surfactants, amphoteric surfactants, dimethicone copolyols, polyether ethoxylates, and mixtures thereof;
   and the oil phase comprises, by weight of the formulation:
(g) about 5% to about 20% by weight of a volatile organic fluid;
(h) about 0.5% to about 2% by weight of a dimethicone copolyol; and
(i) about 5% to about 20% by weight of a linear silicone.

In another prophetic example, the formulation is in the form of a cream, wherein the polar phase comprises, by weight of the formulation:
(a) about 4% to about 30% in total of at least one aluminum-zirconium chlorohydrate salt or complex thereof;
(b) about 0.5% to about 20% in total of at least one betaine component;
(c) about 0.2% to about 10% in total of at least one calcium salt;
(d) about 0.2% to about 10% in total of at least one hydroxy acid; and
(e) about 40% to about 82% of water;
   and the oil phase comprises, by weight of the formulation:
(f) about 2% to about 10% of a volatile organic fluid;
(g) about 0.1% to about 3% of a monoglyceride, diglyceride, triglyceride, or mixture thereof;
(h) about 4% to about 15% in total of at least one cosmetically acceptable surfactant selected cationic surfactants, nonionic surfactants, anionic surfactants, amphoteric surfactants, dimethicone copolyols, polyether ethoxylates, and mixtures thereof; and
(i) about 3% to about 8% in total of at least one C₈₋₂₂ fatty alcohol.

In another prophetic example, the formulation is in the form of a roll-on, wherein the polar phase comprises, by weight of the formulation:
(a) about 4% to about 25% in total of at least one aluminum-zirconium chlorohydrate salt or complex thereof;
(b) about 0.5% to about 20% in total of at least one betaine component;
(c) about 0.2% to about 10% in total of at least one calcium salt;
(d) about 0.2% to about 10% in total of at least one hydroxy acid;
(e) about 30% to about 50% of water;
(f) about 5% to about 40% in total of at least one water miscible solvent; and
(g) 0% to about 2% in total of at least one cosmetically acceptable surfactant selected from cationic surfactants, nonionic surfactants, anionic surfactants, amphoteric surfactants, dimethicone copolyols, polyether ethoxylates, and mixtures thereof;
   and the oil phase comprises, by weight of the formulation:
(h) about 20% to about 50% of a volatile organic or silicone based fluid; and
(i) about 0.5% to about 2% by weight of a dimethicone copolyol.

The antiperspirant and/or deodorant formulation can be provided in any suitable container such as an aerosol can, tube or container with a porous cap, roll-on container, bottle, container with an open end, etc.

A method of the invention for controlling perspiration comprises applying to skin an antiperspirant effective amount of a formulation of any embodiment embraced or specifically described herein.

A method of the invention for controlling odor from perspiration comprises applying to skin a deodorant effective amount of a formulation of any embodiment embraced or specifically described herein.

In a process of the invention for preparing an antiperspirant and/or deodorant composition or formulation, at least one aluminum-zirconium salt or complex thereof is mixed with at least one betaine component in an aqueous medium; and at least one calcium salt and at least one hydroxy acid are added to the resulting mixture to form the composition. Selection of particular aluminum-zirconium, betaine, calcium salt and hydroxy acid components and amounts of these components used can be made in accordance with the disclosure above.

In a process of the invention for stabilizing a composition or formulation comprising an aluminum-zirconium salt or complex thereof, the salt or complex is mixed with at least one betaine component in an aqueous medium; and at least one calcium salt and at least one hydroxy acid are added to the resulting mixture. Selection of particular aluminum-zirconium, betaine, calcium salt and hydroxy acid components and amounts of these components used can be made in accordance with the disclosure above.

Unless stated otherwise, all percentages of composition components given in this specification are by weight based on a total composition or formulation weight of 100%.

Unless otherwise specifically identified, in one embodiment, the ingredients for use in the compositions and formulations of the present invention are cosmetically acceptable ingredients. By "cosmetically acceptable" is meant suitable for use in a formulation for topical application to human skin. A cosmetically acceptable excipient, for example, is an excipient which is suitable for external application in the amounts and concentrations contemplated in the formulations of this invention, and includes for example excipients which are "Generally Recognized as Safe" (GRAS) by the United States Food and Drug Administration.

For purposes of this application, pKa means the pKa in dilute aqueous solution at room temperature and pressure, *e.g*., at ca. 25°C, using standard, art-recognized measuring techniques. For acids that have more than one hydrogen capable of dissociation and so have multiple pKa values, the pKa for purposes of this application refers to the ionization equilibrium with respect to the first hydrogen dissociation step. Thus the pKa of lactic acid for purposes as defined herein would be about 3.86 and glycolic acid would be about 3.83.

The compositions and formulations as provided herein are described and claimed with reference to their ingredients, as is usual in the art. As would be evident to one skilled in the art, the ingredients may in some instances react with one another, so that the true composition of the final formulation may not correspond exactly to the ingredients listed. Thus, it should be understood that the invention extends to the product of the combination of the listed ingredients.

### EXAMPLE

Varying combinations of CaCl₂ and/or hydroxy acids (lactic acid or glycolic acid) are added to a 25% solution of zirconium-aluminum chlorohydrex with betaine (R309; herein "ZAB"). The resulting sample solution is then aged for two months at 45°C before analysis by SEC using a refractive index detector. Table 1 shows data for SEC peaks 1, 3, 4 and 5 for each sample solution. Adjusted area is calculated using total aluminum peak area (sum of peaks 2-5) as the common denominator.

**Table 1: Adjusted SEC peak areas following aging**

| Sample solution | Peak 1 | Peak 3 | Peak 4 | Peak 5 |
|---|---|---|---|---|
| ZAB (R309) | 25.74 | 52.37 | 22.44 | 25.19 |
| ZAB + lactic acid (2%) | 8.57 | 46.24 | 11.58 | 42.18 |
| ZAB + glycolic acid (2%) | 42.32 | 44.66 | 10.01 | 45.32 |
| ZAB + CaCl₂ (2%) | 31.92 | 42.02 | 32.91 | 25.07 |
| ZAB + CaCl₂ (2%) + lactic acid (2%) | 8.80 | 36.62 | 20.22 | 43.16 |
| ZAB + CaCl₂ (2%) + glycolic acid (2%) | 37.86 | 37.99 | 18.92 | 43.09 |

Addition of 2% lactic acid decreases peak 1 area from 25.74% (ZAB alone) to 8.57%, and in presence of CaCl₂ from 31.92% (ZAB + CaCl₂) to 8.80%. This indicates that lactic acid can reduce polymerization of zirconium species. Glycolic acid does not reduce peak 1 area in this study.

Addition of 2% lactic acid increases peak 5 area from 25.19% (ZAB alone) to 42.18%, and in presence of CaCl₂ from 25.07% (ZAB + CaCl₂) to 43.16%. Similarly, addition of 2% glycolic acid increases peak 5 area from 25.19% (ZAB alone) to 45.32%, and in presence of CaCl₂ from 25.07% (ZAB + CaCl₂) to 43.09%. These results indicate that hydroxy acids such as lactic acid and glycolic acid can reduce polymerization of aluminum species.

## Claims

1. An antiperspirant composition comprising a mixing product of:
(a) at least one salt chosen from at least one aluminum salt, at least one aluminum-zirconium salt, at least one aluminum salt complex, and at least one aluminum-zirconium salt complex, wherein the aluminum salt or the aluminum salt complex is at least one salt chosen from aluminum chloride, aluminum chlorohydrate, aluminum sequichlorohydrate, and aluminum dichlorohydrate;
(b) at least one cosmetically acceptable beta-hydroxy acid; and
(c) at least one betaine chosen from 1-carboxy-N,N,N-trimethylmethanaminium hydroxide inner salt and 1-carboxy-N,N,N-trimethylmethanaminium hydroxide inner salt hydrochloride
wherein relative amounts of components (a), (b), and (c) are such that an aqueous solution of these components in the same relative amounts, at a 25% concentration of (a) + (b), following aging of the solution at about 45°C for about 60 days, has an adjusted size exclusion chromatography "SEC" peak 1 area of no more than 40%, an adjusted SEC peak 5 area of at least 30%, and an adjusted SEC peak 4 area of at least 15%, each peak being adjusted based on the total aluminum peak area of the sum of peaks 2 through 5.

2. The composition of claim 1, wherein relative amounts of components (a), (b), and (c) are such that an aqueous solution of these components in the same relative amounts, at a 25% concentration of (a) + (b), following aging of the solution at about 45°C for about 60 days, has an adjusted size exclusion chromatography "SEC" peak 1 area of no more than 40%, an adjusted SEC peak 5 area of at least 30%, and an adjusted SEC peak 4 area of at least 15%.

3. The composition of claim 1, wherein the salt is a chlorohydrate, optionally chosen from aluminum-zirconium pentachlorohydrate, aluminum-zirconium octachlorohydrate, aluminum-zirconium tetrachlorohydrate, aluminum-zirconium trichlorohydrate, and mixtures thereof.

4. The composition of claim 1, wherein the at least one cosmetically acceptable beta-hydroxy acid is chosen from carnitine and salicylic acid.

5. The composition of claim 1, wherein the at least one cosmetically acceptable beta-hydroxy acid comprises salicylic acid.

6. The composition of claim 1 further comprising a divalent metal cation.

7. The composition of claim 6, wherein the divalent metal cation is calcium and a ratio of calcium to metal (Aluminum and Zirconium) on a molar basis is about 0.02 to about 1.2.

8. The composition of claim 1 further comprising a calcium salt chosen from calcium chloride, calcium oxide, and mixtures thereof.

9. The composition of claim 1, wherein the aluminum or aluminum-zirconium salt comprises an aluminum-zirconium chlorohydrate salt, and wherein the metal (Aluminum and Zirconium) to chloride ratio on a molar basis for the salt is about 0.9 to about 2.1, optionally the betaine to zirconium ration on a molar basis is about 0.1 to about 2 and/or optionally the betaine to hydroxy acid ratio of a molar basis is about 0.2 to about 30.

10. The composition of claim 1, wherein the composition comprises less than 1% by weight of an amino acid.

11. The composition of claim 2, which when analyzed by size exclusion chromatography as a 5% aqueous solution using conditions capable of resolving the aluminum and/or zirconium species into at least 4 successive peaks, the ratio of the SEC area of the first major peak to the SEC area of the last major peak is less than about 1, optionally less than about 0.25.

12. A composition comprising the stabilized antiperspirant composition of claim 1 in combination with a cosmetically acceptable carrier.

13. A method for controlling perspiration comprising applying to skin a formulation comprising an antiperspirant effective amount of the composition of claim 1.

14. A process for preparing or stabilizing the antiperspirant composition of claim 1 comprising:
i. mixing the at least one salt with the at least one betaine component in an aqueous medium; and
ii. adding to the resulting mixture at least one hydroxy acid to form the composition.

15. The composition of claim 2, wherein the SEC peak 1 area is no more than 10%.

16. The composition of claim 2, wherein the SEC peak 5 area is at least 40%.

17. The composition of claim 2, wherein the SEC peak 1 area is no more than 10% and the SEC peak 5 area is at least 40%.
